# EUROPÄISCHE PATENTSCHRIFT

(11) **EP 0 785 185 B1**
(45) Veröffentlichungstag und Bekanntmachung des Hinweises auf die Patenterteilung: **08.12.1999**
(21) Anmeldenummer: 97100242.3
(22) Anmeldetag: 09.01.1997
(51) Int. Cl.: C07C 209/60

(54) **Verfahren zur Herstellung von Aminen aus Olefinen an kristallinen Oxiden auf der Basis von Alumophosphaten mit Faujasit-Struktur**
Process for the preparation of amines from olefins with crystalline oxides based on aluminophosphates having the structure of faujasite
Procédé de préparation d'amines à partir d'oléfines avec des oxydes cristallins basés sur des aluminophosphates possédant la structure de la faujasite

(30) Priorität: 17.01.1996 DE 19601409
(43) Veröffentlichungstag der Anmeldung: 23.07.1997
(73) Patentinhaber: BASF AKTIENGESELLSCHAFT, 67056 Ludwigshafen (DE)
(72) Erfinder: Eller, Karsten, Dr., 67059 Ludwigshafen (DE); Kummer, Rudolf, Dr., 67227 Frankenthal (DE); Stops, Peter, 67122 Altrip (DE)

(56) Entgegenhaltungen:
- EP-A- 0 752 409
- US-A- 4 375 002
- PATENT ABSTRACTS OF JAPAN vol. 016, no. 411 (C-0979), 31.August 1992 & JP 04 139156 A (MITSUI TOATSU CHEM INC), 13.Mai 1992,
- DATABASE WPI Week 9513 Derwent Publications Ltd., London, GB; AN 95-096945 XP002030527 & RU 2 015 959 C (AVETISOV A.K.) , 15.Juli 1994

## Beschreibung

Die vorliegende Erfindung betrifft ein Verfahren zur Herstellung von Aminen durch Umsetzung von Ammoniak oder primären oder sekundären Aminen mit Olefinen bei erhöhten Temperaturen und Drükken in Gegenwart von kristallinen Oxiden auf der Basis von Alumophosphaten mit Faujasit-Struktur.

Eine Übersicht über die Methoden zur Aminierung von Olefinen wird in "Functionalisation of Alkenes: Catalytic Amination of Monoolefins", J.J. Brunet et al. J.Mol.Catal., 49 (1989), Seiten 235 bis 259 gegeben.

Grundsätzlich gibt es zwei Katalysemechanismen. Das Olefin wird über einen Metallkomplex koordiniert. Diese aktivierte Spezies kann von dem nucleophilen Amin angegriffen werden und ein höher aminiertes Produkt bilden. Das Amin kann an Säurezentren oder an Metallzentren (via Metallamide) chemisorbiert werden und so aktiviert mit dem Olefin umgesetzt werden.

Gut geeignete Katalysatoren sind Zeolithe. Sie zeichnen sich durch eine hohe Anzahl an katalytisch aktiven Zentren aus, kombiniert mit einer großen Oberfläche. Die beschriebenen Zeolithe unterscheiden sich im Typ und in der Nachbehandlung (z.B. thermische Behandlung, Dealuminierung, Säurebehandlung, Metallioneneintausch, etc.). Beispiele hierfür finden sich in US-A-4 375 002, US-A-4 536 602, EP-A-305 564, EP-A-101 921, DE-A-42 06 992.

Aus EP-A-133 938, EP-A-431 451 und EP-A-132 736 sind Verfahren bekannt, bei denen Bor-, Gallium-, Alumino- und Eisensilikatzeolithe für die Herstellung von Aminen aus Olefinen verwendet werden und auf die Möglichkeit der Dotierung dieser Zeolithe mit Alkali-, Erdalkali- und Übergangsmetallen hingewiesen wird.

Aus CA-A-2 092 964 ist ein Verfahren zur Herstellung von Aminen aus Olefinen bekannt, bei dem BETA-Zeolithe, die als kristalline Aluminosilikate bestimmter Zusammensetzung mit einer Porengröße von mehr als 5 Å definiert sind, eingesetzt werden. Bevorzugt werden metall- oder halogenmodifizierte Beta-Zeolithe eingesetzt.

Aus JP-A-04/139156 ist ein Verfahren bekannt, um tertiäre Amine aus Olefinen an Silico-Alumophosphaten oder Metallo-Silicoalumophosphaten herzustellen. Die Verwendung von Diisopropylamin als Templat für die Synthese der kristallinen SAPOs oder MeAPOs führt jedoch meist zu kristallinen Strukturen mit relativ engen Porendurchmessern (Handbook of Molecular Sieves, Van Nostrand Reinhold, New York, 1992, Seite 578), z.B. vom Typ CoAPO-11 (AEL), CoAPO-34 (CHA) oder MgAPSO-39 (ATN). Einzig SAPO-5 (AFI) besitzt Porendurchmesser, in die beispielsweise Isobuten als kleinstes Olefin hineindiffundieren kann, um mit Ammoniak die tertiären Amine zu bilden.

Alle Verfahren zur Synthese von Aminen aus Olefinen an diesen Katalysatoren zeichnen sich durch eine geringe Amin-Ausbeute oder geringe Raum-Zeit-Ausbeute aus, oder führen zu einer raschen Desaktivierung der Katalysatoren.

Ferner ist aus EP-A-752 409 ein Verfahren zur Herstellung von Aminen aus Gemischen, die man bei der Spaltung von Erdölfraktionen erhält, in Gegenwart verschiedener Heterogenkatalysatoren bekannt.

Aus RU-A-2015959 ist die Verwendung eines amorphen Alumophosphates als Katalysator zur Herstellung von Diphenylamin aus zwei Molekülen Anilin unter Abspaltung von Ammoniak bekannt.

Der vorliegenden Erfindung lag daher die Aufgabe zugrunde, diesen Nachteilen abzuhelfen.

Überraschend wurde gefunden, daß mit kristallinen Oxiden auf der Basis von Alumophosphaten mit Faujasit-Struktur den oben genannten Nachteilen abgeholfen werden kann. Diese Katalysatoren galten als nicht einsetzbar, da sie sich an Luft zu amorphem Material umwandeln. Durch Calcinierung der Templat-haltigen Kristalle direkt im Aminierungsreaktor gelang es jedoch, sie für den gewünschten Zweck einzusetzen, ohne daß sie sich zu den weniger aktiven amorphen Alumophosphaten umwandelten.

Demgemäß wurde ein neues und verbessertes Verfahren zur Herstellung von Aminen der allgemeinen Formel I 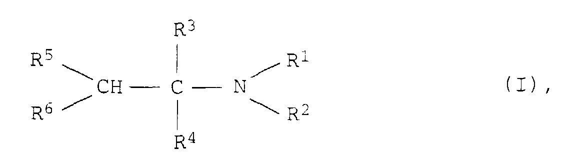 in der
- R¹,R²,R³,R⁴,R⁵,R⁶: Wasserstoff, C₁- bis C₂₀-Alkyl, C₂- bis C₂₀-Alkenyl, C₂- bis C₂₀-Alkinyl, C₃- bis C₂₀-Cycloalkyl, C₄- bis C₂₀-Alkyl-cycloalkyl, C₄- bis C₂₀-Cycloalkyl-alkyl, Aryl, C₇- bis C₂₀-Alkylaryl oder C₇- bis C₂₀-Aralkyl,
- R¹ und R²: gemeinsam eine gesättigte oder ungesättigte C₃- bis C₉-Alkylendikette und
- R³ oder R⁵: C₂₁- bis C₂₀₀-Alkyl, C₂₁- bis C₂₀₀-Alkenyl oder gemeinsam eine C₂- bis C₁₂-Alkylendikette
bedeuten, durch Umsetzung von Olefinen der allgemeinen Formel II 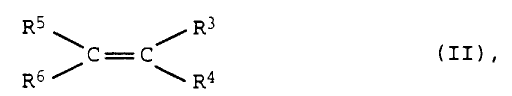 in der R³, R⁴, R⁵ und R⁶ die oben genannten Bedeutungen haben, mit Ammoniak oder primären oder sekundären Aminen der allgemeinen Formel III 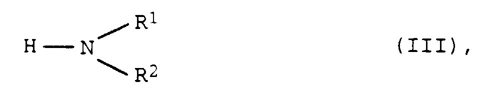 in der R¹ und R² die oben genannten Bedeutungen haben, bei Temperaturen von 200 bis 350°C und Drücken von 100 bis 300 bar in Gegenwart eines Heterogenkatalysators gefunden, welches dadurch gekennzeichnet ist, daß man als Heterogenkatalysator kristalline Oxide auf der Basis von Alumophosphaten mit Faujasit-Struktur einsetzt.

Das erfindungsgemäße Verfahren kann wie folgt durchgeführt werden:

Das Olefin II und Ammoniak oder das primäre oder sekundären Amin III kann bei Temperaturen von 200 bis 350°C, bevorzugt 220 bis 330°C, besonders bevorzugt 230 bis 320°C und Drücken von 100 bis 300 bar, bevorzugt 120 bis 300 bar, besonders bevorzugt 140 bis 290 bar in Gegenwart von kristallinen Oxiden auf der Basis von Alumophosphaten mit Faujasit-Struktur als Katalysator z.B. in einem Druck-Reaktor umgesetzt werden, und bevorzugt das erhaltene Amin abgetrennt und die nichtumgesetzten Einsatzstoffe zurückführt werden.

Das vorliegende Verfahren zeichnet sich durch eine sehr gute Ausbeute bei hoher Selektivität und bei hoher Raum-Zeit-Ausbeute aus. Zudem ist die Desaktivierung des Katalysators zurückgedrängt worden.

Das erfindungsgemäße Verfahren zeichnet sich dadurch aus, daß bereits mit niedrigem Ammoniak- bzw. Amin- Überschuß eine hohe Selektivität an gewünschtem Reaktionsprodukt erzielt und die Dimerisierung und/oder Oligomerisierung des eingesetzten Olefins vermieden wird.

Eine Ausführungsform dieses Verfahrens besteht darin, daß man Ammoniak und/oder Amine III zusammen mit dem Olefin II im molaren Verhältnis von 1:1 bis 5:1 gemischt einem Festbettreaktor zuführt und bei einem Druck von 100 bis 300 bar und einer Temperatur von 200 bis 350°C in der Gasphase oder im überkritischen Zustand umsetzt.

Aus dem Reaktionsaustrag kann das gewünschte Produkt mit Hilfe bekannter Methoden, beispielsweise Destillation oder Extraktion, erhalten und nötigenfalls mittels weiterer Trennoperationen auf die gewünschte Reinheit gebracht werden. Die nichtumgesetzten Eingangsstoffe werden in der Regel bevorzugt in den Reaktor zurückgeführt.

Man kann einfach oder mehrfach ungesättigte Olefine II, insbesondere solche mit 2 bis 10 C-Atomen bzw. deren Mischungen und Polyolefine als Ausgangsstoffe verwenden. Wegen der geringer ausgeprägten Polymerisationsneigung eignen sich Monoolefine besser als Di- und Polyolefine, doch können diese mit Hilfe höherer Ammoniak- bzw. Aminüberschüsse ebenso selektiv umgesetzt werden. Die Lage des Gleichgewichts und damit der Umsatz zum gewünschten Amin ist sehr stark vom gewählten Reaktionsdruck abhängig. Hoher Druck begünstig das Additionsprodukt, doch stellt im allgemeinen aus technischen und wirtschaftlichen Gründen der Druckbereich bis 300 bar das Optimum dar. Die Selektivität der Reaktion wird - neben Größen wie Ammoniak-/Amin-Überschuß und Katalysator - in hohem Maß durch die Temperatur beeinflußt. Zwar nimmt die Reaktionsgeschwindigkeit der Additionsreaktion mit steigender Temperatur stark zu, doch werden konkurrierende Crack- und Rekombinationsreaktionen des Olefins gleichzeitig gefördert. Zudem ist eine Temperaturerhöhung aus thermodynamischer Sicht nicht vorteilhaft. Die Lage des Temperaturoptimums bezüglich Umsatz und Selektivität ist von der Konstitution des Olefins, des eingesetzten Amins und des Katalysators abhängig und liegt meist im Bereich von 200 bis 350°C.

Als Katalysatoren für die Aminierung von Olefinen eignen sich kristalline Oxide auf Basis von Alumophosphaten mit Faujasit-Struktur. Derartige Materialien sind beispielsweise aus US-A-4 440 871 als SAPO-37, oder aus EP-A-158 976 als FCAPO-37 oder aus EP-A-158 977 als MeAPO-37 bekannt. Eine Diskussion der strukturellen Zusammenhänge von SAPOs, AlPOs, MeAPOs und MeAPSOs findet sich z.B. in Stud. Surf. Sci. Catal. 37 (1987) Seite 13 bis 27. Die erfindungsgemäßen AlPOs enthalten Aluminium und Phosphor im Verhältnis größer 1, in den SAPOs ist ein Teil des Phosphors und/oder gleichzeitig Aluminium und Phosphor durch Silicium ersetzt, in den MeAPOs kommen neben Aluminium und Phosphor noch verschiedene Metallionen wie beispielsweise Li, B, Be, Mg, Ti, Mn, Fe, Co, Zn, Ga, Ge, As vor, bei den MeAPSOs zusätzlich noch Silicium. Die negative Ladung des MeₐAl_{b}P_{c}Si_{d}Oₑ-Gerüstes wird durch Kationen kompensiert.

Die erfindungsgemäßen kristallinen Oxiden auf der Basis von Alumophosphaten mit Faujasit-Struktur können als solche, oder aber auch mit einem Bindemittel im Verhältnis 98:2 bis 40:60 Gew.-% zu Strängen oder Tabletten verformt werden. Als Bindemittel eignen sich verschiedene Aluminiumoxide, bevorzugt Boehmit, amorphe Aluminosilikate mit einem SiO₂/Al₂O₃-Verhältnis von 25:75 bis 95:5, Siliciumdioxid, bevorzugt hochdisperses SiO₂, Gemische aus hochdispersem SiO₂ und hochdispersem Al₂O₃, hochdisperses TiO₂ sowie Tone. Nach der Verformung werden die Extrudate oder Preßlinge zweckmäßig bei 110°C/16 h getrocknet und bei 200 bis 500°C/2 bis 16 h calciniert, wobei die Calcinierung vorzugsweise direkt (in situ) im Aminierungsreaktor erfolgt.

Zur Erhöhung der Selektivität, der Standzeit und der Anzahl der möglichen Regenerierungen kann man verschiedene Modifizierungen an den erfindungsgemäßen kristallinen Oxiden auf der Basis von Alumophosphaten mit Faujasit-Struktur vornehmen.

Eine Modifizierung der Katalysatoren besteht darin, daß man die kristallinen Oxiden auf der Basis von Alumophosphaten mit Faujasit-Struktur mit Alkalimetallen wie Na und K, Erdalkalimetallen wie Ca, Mg, Erdmetallen wie Tl, Übergangsmetallen wie z.B. Ti, Zr, Mn, Fe, Mo, Cu, Zn, Cr, Edelmetallen und/oder seltenen Erdmetallen wie z.B. La, Ce, Y dotieren kann.

Die Katalysatoren kann man als Stränge mit Durchmessern von z.B. 1 bis 4 mm oder als Tabletten mit z.B. 3 bis 5 mm Durchmesser für die Aminierung der Olefine einsetzen.

Aus dem beispielsweise zu Strängen verformten Katalysator kann man durch Mahlen und Sieben ein Wirbelgut in der Größe von 0,1 bis 0,8 mm erhalten.

Die Substituenten R¹, R², R³, R⁴, R⁵ und R⁶ in den Verbindungen I, II und III haben folgende Bedeutungen:
R¹,R²,R³,R⁴,R⁵,R⁶
   - Wasserstoff,
   - C₁- bis C₂₀-Alkyl, bevorzugt C₁- bis C₁₂-Alkyl, besonders bevorzugt C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, n-Hexyl, iso-Hexyl, n-Heptyl, iso-Heptyl, n-Octyl und iso-Octyl,
   - C₂- bis C₂₀-Alkenyl, bevorzugt C₂- bis C₁₂-Alkenyl, besonders bevorzugt C₂- bis C₈-Alkenyl wie Vinyl und Allyl,
   - C₂- bis C₂₀-Alkinyl, bevorzugt C₂- bis C₈-Alkinyl, insbesondere C₂H und Propargyl,
   - C₃- bis C₂₀-Cycloalkyl, bevorzugt C₃- bis C₁₂-Cycloalkyl, besonders bevorzugt C₅- bis C₈-Cycloalkyl wie Cyclopentyl, Cyclohexyl, Cycloheptyl und Cyclooctyl,
   - C₄- bis C₂₀-Alkyl-cycloalkyl, bevorzugt C₄- bis C₁₂-Alkyl-cycloalkyl, besonders bevorzugt C₅- bis C₁₀-Alkyl-cycloalkyl,
   - C₄- bis C₂₀-Cycloalkyl-alkyl, bevorzugt C₄- bis C₁₂-cycloalkyl-alkyl, besonders bevorzugt C₅- bis C₁₀-Cycloalkyl-alkyl,
   - Aryl wie Phenyl, 1-Naphthyl und 2-Naphthyl, bevorzugt Phenyl,
   - C₇- bis C₂₀-Alkylaryl, bevorzugt C₇- bis C₁₆-Alkylaryl, bevorzugt C₇- bis C₁₂-Alkylphenyl wie 2-Methylphenyl, 3-Methylphenyl, 4-Methylphenyl, 2-Ethylphenyl, 3-Ethylphenyl und 4-Ethylphenyl,
   - C₇- bis C₂₀-Aralkyl, bevorzugt C₇- bis C₁₆-Aralkyl, bevorzugt C₇- bis C₁₂-Phenalkyl wie Phenylmethyl, 1-Phenylethyl, 2-Phenylethyl,
R¹ und R²
   - gemeinsam eine gesättigte oder ungesättigte C₃- bis C₉-Alkylendikette, bevorzugt- -(CH₂)₄-, -(CH₂)₅-, -(CH₂)₇- und -CH=CH-CH=CH-,
R³ oder R⁵
   - C₂₁- bis C₂₀₀-Alkyl, bevorzugt C₄₀- bis C₂₀₀-Alkyl, wie Polybutyl, Polyisobutyl, Polypropyl, Polyisopropyl und Polyethyl, besonders bevorzugt Polybutyl und Polyisobutyl,
   - C₂₁- bis C₂₀₀-Alkenyl, bevorzugt C₄₀- bis C₂₀₀-Alkenyl, besonders bevorzugt C₇₀- bis C₁₇₀-Alkenyl,
R³ und R⁵
   - gemeinsam eine C₂- bis C₁₂-Alkylendikette, bevorzugt eine C₃- bis C₈-Alkylendikette, besonders bevorzugt -(CH₂)₃-, -(CH₂)₄-, -(CH₂)₅-, - -(CH₂)₆- und - (CH₂)₇-, insbesondere -(CH₂)₃- und -(CH₂)₄-.

### Beispiele

### Katalysatorsynthesen

### Katalysator A: SAPO-37

Lösung A wurde hergestellt aus 152,5 g H₂O, 138,5 g 85%-iger H₃PO₄ und 83 g Pural® SB (AlOOH, der Firma Condea) und für 6 Stunden bei Raumtemperatur gerührt.

Lösung B wurde hergestellt aus 580 g einer 40%-igen Tetrapropylammoniumhydroxidlösung, 5,5 g Tetramethylammoniumhydroxid Pentahydrat und 33,5 g Aerosil® 200 (SiO₂, der Firma Degussa) und für 2 Stunden bei Raumtemperatur gerührt.

Lösung B wurde unter Rühren langsam zu Lösung A gegeben und 24 Stunden bei Raumtemperatur gerührt. Die Mischung wurde in einen 2 Liter Autoklaven überführt und 48 Stunden bei 200°C unter Eigendruck von 17 bar kristallisiert. Die gebildeten SAPO-37 Kristalle wurden abzentrifugiert und 16 Stunden bei 110°C getrocknet. Ausbeute: 84 g eines nach Röntgendiffraktrometrie phasenreinen SAPO-37. 60 g SAPO-37 wurden mit 40 g Boehmit, 2 g Ameisensäure und 3 g Stärke versetzt, im Kneter kompaktiert und unter Wasserzusatz (75 ml) 60 min verknetet. In einer Strangpresse wurden mit einem Pressdruck von 50 bar 2 mm Stränge erzeugt und diese 16 Stunden bei 110°C getrocknet. 18 g dieser Templat-haltigen Stränge wurden als Splitt in den Reaktor eingebaut (entsprechend 14 g H-SAPO-37) und mit 20 Liter trockener Luft pro Stunde calciniert. Es wurde ein Temperaturprogramm von 2 Stunden 350°C, 2 Stunden 450°C und 8 Stunden 490°C gefahren.

### Katalysator B: SAPO-5 (Vergleichsbeispiel)

64 g SAPO-5 wurden mit 54 g eines 30%-igen Kieselsols und 5 g Stärke versetzt, im Kneter kompaktiert und unter Wasserzusatz (16 ml) 45 min verknetet. In einer Strangpresse wurden mit einem Pressdruck von 100 bar 2 mm Stränge erzeugt und diese 16 Stunden bei 110°C getrocknet und 16 Stunden bei 500°C calciniert.

### Katalysator C: HY-Zeolith (Vergleichsbeispiel)

2160 g NaY wurden mit 1440 g Boehmit und 72 g Ameisensäure versetzt, im Kneter kompaktiert und unter Wasserzusatz (1850 ml) 60 min verknetet. In einer Strangpresse wurden mit einem Pressdruck von 90 bar 2 mm Stränge erzeugt und diese 16 Stunden bei 110°C getrocknet und 16 Stunden bei 500°C calciniert. Die fertigen Stränge wurden viermal mit 20%-iger NH₄Cl-Lösung bei 80°C ionengetauscht und schließlich 5 Stunden bei 500°C calciniert.

### Katalysator D: Amorphes Produkt aus H-SAPO-37 (Vergleichsbeispiel)

H-SAPO-37 wurde durch Calcinieren von Katalysator A erhalten und an Luft gehandhabt. Aus der Röntgendiffraktometrie ergab sich, daß das Material amorph vorlag.

### Katalysator E: SAPO-37

Katalysator E wurde analog zu Katalysator A hergestellt, aber die Synthese erfolgte in einem gerührten 2,5 l Autoklaven. Es wurden 38 g eines gut kristallinen SAPO-37 Pulvers erhalten, die analog zu Katalysator A verstrangt wurden und in situ calciniert wurden.

### Katalysator F: SAPO-11 (Vergleichsbeispiel)

122,7 g Aluminiumisopropylat und 15 g dest. H₂O wurden unter Rühren mit 69,4 g 85 %iger H₃PO₄ in 180 g dest. H₂O versetzt. 13,6 g Ludox® AS 40 (40 % SiO₂) in 20 g dest. H₂O und 30,4 g Diisopropylamin in 15 g dest. H₂O wurden hinzugefügt und die Mischung anschließend in einem Autoklaven 48 h bei 200°C kristallisiert. Die gebildeten SAPO-11 Kristalle wurden abfiltriert, gewaschen und 4 h bei 120°C getrocknet sowie 12 h bei 500°C calciniert.

Ausbeute: 70 g eines nach Röntgendiffraktrometrie phasenreinen SAPO-11.

60 g SAPO-11 wurden mit 40 g Boehmit und 2 g Ameisensäure versetzt, im Kneter kompaktiert und unter Wasserzusatz (53 ml) 45 min verknetet. In einer Strangpresse wurden mit einem Preßdruck von 70 bar 2 mm Stränge erzeugt und diese 16 h bei 120° getrocknet sowie 16 h bei 500° calciniert.

### Aminierungsbeispiele

Die Versuche wurden in einem Rohrreaktor (6 mm Innendurchmesser) unter isothermen Bedingungen bei 270 bis 300°C und einem Druck von 280 bar mit einem Gemisch aus Isobuten und Ammoniak im molaren Verhältnis von 1:1,5 durchgeführt. Die Reaktionsprodukte wurden im Gaschromatographen analysiert.

Die Ergebnisse sind in Tabelle 1 zusammengestellt und zeigen, daß die erfindungsgemäßen Katalysatoren höhere Ausbeuten als die bisher bekannten Katalysatorsysteme SAPO-5 aufweisen, daß insbesondere bei den technisch wichtigen höheren Belastungen größere Ausbeuten als mit anderen Faujasiten aus der Klasse der Alumosilikate erzielt werden und daß amorphe Oxide mit einer erfindungsgemäßen Zusammensetzung ebenfalls geringere Ausbeuten ergeben.

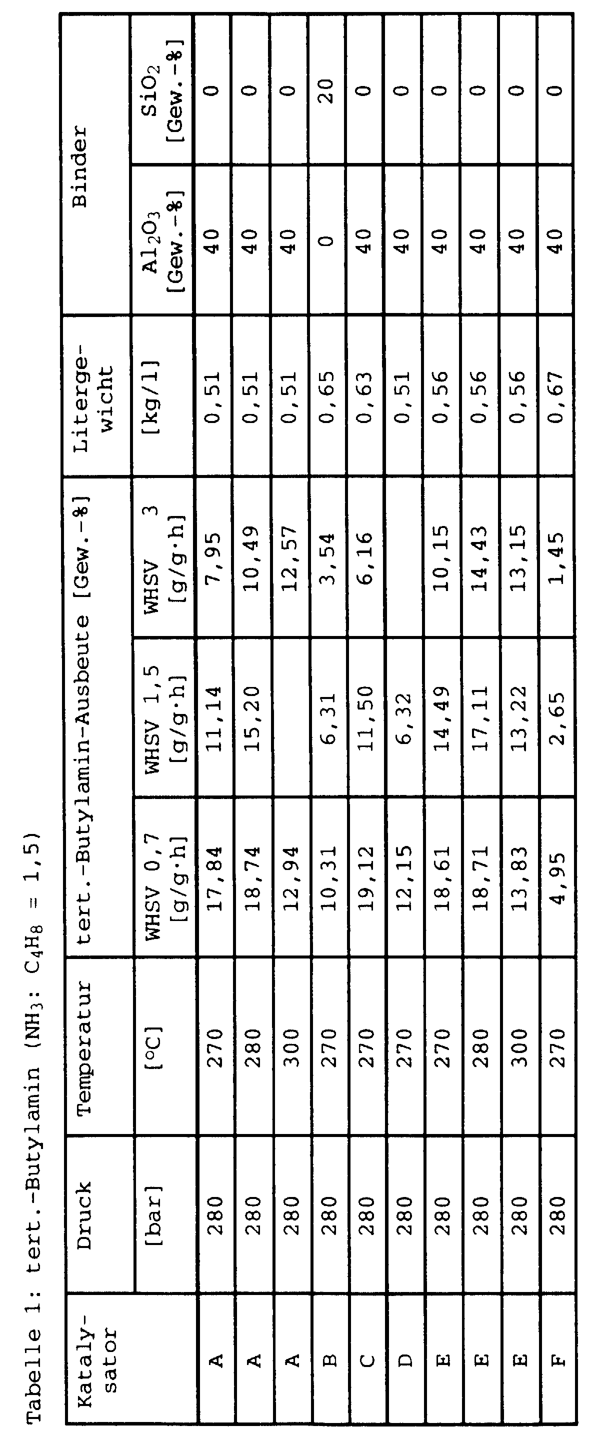

## Patentansprüche

1. Verfahren zur Herstellung von Aminen der allgemeinen Formel I 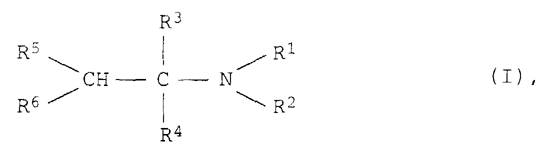 in der
R¹,R²,R³,R⁴,R⁵,R⁶ Wasserstoff, C₁- bis C₂₀-Alkyl, C₂- bis C₂₀-Alkenyl, C₂- bis C₂₀-Alkinyl, C₃- bis C₂₀-Cycloalkyl, C₄- bis C₂₀-Alkyl-cycloalkyl, C₄- bis C₂₀-Cycloalkyl-alkyl, Aryl, C₇- bis C₂₀-Alkylaryl oder C₇- bis C₂₀-Aralkyl,
R¹ und R² gemeinsam eine gesättigte oder ungesättigte C₃- bis C₉-Alkylendikette und
R³ oder R⁵ C₂₁- bis C₂₀₀-Alkyl, C₂₁- bis C₂₀₀-Alkenyl oder gemeinsam eine C₂- bis C₁₂-Alkylendikette
bedeuten, durch Umsetzung von Olefinen der allgemeinen Formel II 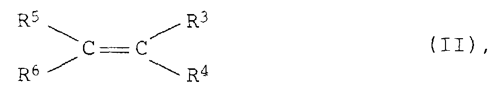 in der R³, R⁴, R⁵ und R⁶ die oben genannten Bedeutungen haben, mit Ammoniak oder primären oder sekundären Aminen der allgemeinen Formel III 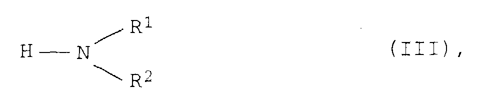 in der R¹ und R² die oben genannten Bedeutungen haben, bei Temperaturen von 200 bis 350°C und Drücken von 100 bis 300 bar in Gegenwart eines Heterogenkatalysators, dadurch gekennzeichnet, daß man als Heterogenkatalysator kristalline Oxide auf der Basis von Alumophosphaten mit Faujasit-Struktur einsetzt, die im Reaktor (in situ) durch Calcinierung der templathaltigen Form erzeugt werden.

2. Verfahren zur Herstellung von Aminen 1 nach Anspruch 1, dadurch gekennzeichnet, daß man das gebildete Amin 1 abtrennt und die nichtumgesetzten Einsatzstoffe II und III zurückführt.

3. Verfahren zur Herstellung von Aminen nach den Ansprüchen 1 bis 2, dadurch gekennzeichnet, daß man als Olefin II Isobuten, Diisobuten, Cyclopenten, Cyclohexen oder Polyisobuten einsetzt.

4. Verfahren zur Herstellung von Aminen nach den Ansprüchen 1 bis 3, dadurch gekennzeichnet, daß man als Heterogenkatalysatoren kristalline Oxide auf der Basis von Alumophosphaten mit Faujasit-Struktur in der H-Form oder in der Ammoniumform einsetzt.

5. Verfahren zur Herstellung von Aminen nach den Ansprüchen 1 bis 4, dadurch gekennzeichnet, daß man als Heterogenkatalysatoren kristalline Oxide auf der Basis von Alumophosphaten mit Faujasit-Struktur, die mit einem oder mehreren Übergangsmetallen dotiert sind, einsetzt.

6. Verfahren zur Herstellung von Aminen nach den Ansprüchen 1 bis 5, dadurch gekennzeichnet, daß man als Heterogenkatalysatoren kristalline Oxide auf der Basis von Alumophosphaten mit Faujasit-Struktur, die mit einem oder mehreren Elementen der Seltenen Erden dotiert sind, einsetzt.

7. Verfahren zur Herstellung von Aminen nach den Ansprüchen 1 bis 6, dadurch gekennzeichnet, daß man als Heterogenkatalysatoren kristalline Oxide auf der Basis von Alumophosphaten mit Faujasit-Struktur einsetzt, die mit einem oder mehreren Elementen aus der Gruppe der Alkali-, Erdalkalimetalle oder Erdmetalle dotiert sind.

8. Verfahren zur Herstellung von Aminen nach den Ansprüchen 1 bis 7, dadurch gekennzeichnet, daß man als Heterogenkatalysatoren kristalline Oxide auf der Basis von Alumophosphaten mit Faujasit-Struktur einsetzt, die mit einem Bindemittel verformt und bei Temperaturen von 200 bis 600°C calciniert sind.

9. Verfahren zur Herstellung von Aminen nach den Ansprüchen 1 bis 8, dadurch gekennzeichnet, daß man als Heterogenkatalysatoren Silicoalumophosphate (SAPOs), insbesondere SAPO-37 oder FCAPO-37, Metallo-Silicoalumophosphate (MeAPSOs), insbesondere MeAPSO-37, Alumophosphate (AlPOs) mit Aluminiumüberschuß, insbesondere AlPO-37 oder Metallo-Alumophosphate (MeAPOs), insbesondere MeAPO-37, einsetzt.

10. Verfahren zur Herstellung von Aminen nach Anspruch 9, dadurch gekennzeichnet, daß man als Heterogenkatalysatoren SAPO-37, FCAPO-37, AIPO-37 oder MeAPO-37 einsetzt.

## Claims

1. A process for preparing amines of the general formula I 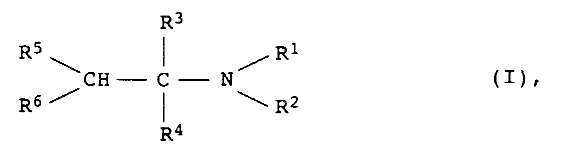 where
R¹,R²,R³,R⁴,R⁵ and R⁶ are each hydrogen, C₁-C₂₀-alkyl, C₂-C₂₀-alkenyl, C₂-C₂₀-alkynyl, C₃-C₂₀-cycloalkyl, C₄-C₂₀-alkylcycloalkyl, C₄-C₂₀-cycloalkylalkyl, aryl, C₇-C₂₀-alkylaryl or C₇-C₂₀-aralkyl,
R¹ and R² are together a saturated or unsaturated C₃-C₉-alkylene dichain, and
R³ or R⁵ are each C₂₁-C₂₀₀-alkyl or C₂₁-C₂₀₀-alkenyl or together a C₂-C₁₂-alkylene dichain,
by reacting olefins of the general formula II 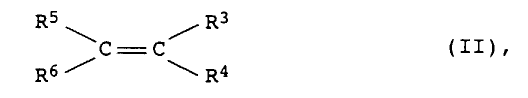 where R³, R⁴, R⁵ and R⁶ are each as defined above, with ammonia or primary or secondary amines of the general formula III 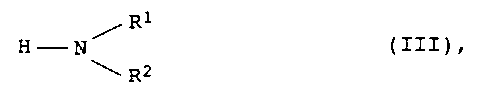 where R¹ and R² are each as defined above, at temperatures from 200 to 350°C and pressures from 100 to 300 bar in the presence of a heterogeneous catalyst, characterized in that the heterogeneous catalyst is selected from crystalline oxides based on faujasite alumophosphates and generated in situ in the reactor by calcination of the template-containing form.

2. A process as claimed in claim 1, characterized in that the product amine I is separated off and the unconverted feed materials II and III are recycled.

3. A process as claimed in claim 1 or 2, characterized in that olefin II is isobutene, diisobutene, cyclopentene, cyclohexene or polyisobutene.

4. A process as claimed in any of claims 1 to 3, characterized in that the heterogeneous catalyst is selected from crystalline oxides based on faujasite alumophosphates and used in the H-form or in the ammonium form.

5. A process as claimed in any of claims 1 to 4, characterized in that the heterogeneous catalyst is selected from crystalline oxides based on faujasite alumophosphates and doped with one or more transition metals.

6. A process as claimed in any of claims 1 to 5, characterized in that the heterogeneous catalyst is selected from crystalline oxides based on faujasite alumophosphates and doped with one or more rare earth elements.

7. A process as claimed in any of claims 1 to 6, characterized in that the heterogeneous catalyst is selected from crystalline oxides based on faujasite alumophosphates and doped with one or more elements from the group of the alkali, alkaline earth or earth metals.

8. A process as claimed in any of claims 1 to 7, characterized in that the heterogeneous catalyst is selected from crystalline oxides based on faujasite alumophosphates and molded with a binder and calcined at from 200 to 600°C.

9. A process as claimed in any of claims 1 to 8, characterized in that the heterogeneous catalyst is selected from silicoalumophosphates (SAPOs), in particular SAPO-37 or FCAPO-37, metallo-silicoalumophosphates (MeAPSOs), in particular MeAPSO-37, alumophosphates (AlPOs) having an excess of aluminum, in particular AIPO-37, or metallo-alumophosphates (MeAPOs), in particular MeAPO-37.

10. A process as claimed in claim 9, characterized in that the heterogeneous catalyst is selected from SAPO-37, FCAPO-37, AlPO-37 or MeAPO-37.

## Revendications

1. Procédé pour la préparation d'amines de formule générale I 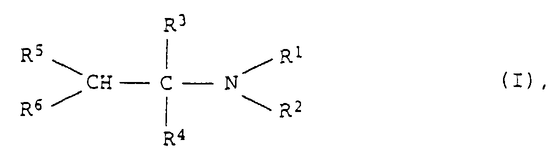 dans laquelle
R¹, R², R³, R⁴, R⁵, R⁶ représentent chacun l'hydrogène, un groupe alkyle en C1-C20, alcényle en C2-C20, alcynyle en C2-C20, cycloalkyle en C3-C20, alkylcycloalkyle en C4-C20, cycloalkylealkyle en C4-C20, aryle, alkylaryle en C7-C20 ou aralkyle en C7-C20,
R¹ et R² représentent ensemble une chaîne divalente alkylène saturée ou insaturée en C3-C9 et
R³ ou R⁵ représentent un groupe alkyle en C21-C200, alcényle en C21-C200 ou bien R³ et R⁵ représentent ensemble une chaîne divalente alkylène en C2-C12,
par réaction d'oléfines de formule générale II 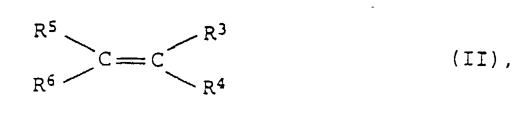 dans laquelle R³, R⁴, R⁵ et R⁶ ont les significations indiquées ci-dessus, avec l'ammoniac ou des amines primaires ou secondaires de formule générale III 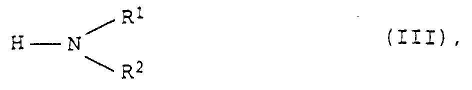 dans laquelle R¹ et R² ont les significations indiquées ci-dessus, à des températures de 200 à 350°C et des pressions de 100 à 300 bar en présence d'un catalyseur hétérogène, caractérisé par le fait que l'on utilise en tant que catalyseurs hétérogènes des oxydes cristallins à base d'aluminophosphates à structure de faujasite qu'on forme dans le réacteur (in situ) par calcination de la forme comportant une matrice.

2. Procédé pour préparer des amines I selon la revendication 1, caractérisé par le fait que l'on sépare l'amine I formée et on recycle les produits de départ II et III non convertis.

3. Procédé pour la préparation d'amines selon les revendications 1 à 2, caractérisé par le fait que l'oléfine mise en oeuvre II est l'isobutène, le diisobutène, le cyclopentène, le cyclohexène ou un polyisobutène.

4. Procédé pour la préparation d'amines selon les revendications 1 à 3, caractérisé par le fait que l'on utilise en tant que catalyseurs hétérogènes des oxydes cristallins à base d'aluminophosphates à structure de faujasite sous la forme H ou sous la forme ammonium.

5. Procédé pour la préparation d'amines selon les revendications 1 à 4, caractérisé par le fait que l'on utilise en tant que catalyseurs hétérogènes des oxydes cristallins à base d'aluminophosphates à structure de faujasite dopés par un ou plusieurs métaux de transition.

6. Procédé pour la préparation d'amines selon les revendications 1 à 5, caractérisé par le fait que l'on utilise en tant que catalyseurs hétérogènes des oxydes cristallins à base d'aluminophosphates à structure de faujasite dopés par un ou plusieurs éléments des terres rares.

7. Procédé pour la préparation d'amines selon les revendications 1 à 6, caractérisé par le fait que l'on utilise en tant que catalyseurs hétérogènes des oxydes cristallins à base d'aluminophosphates à structure de faujasite dopés par un ou plusieurs éléments du groupe des métaux alcalins, des métaux alcalino-terreux ou des métaux terreux.

8. Procédé pour la préparation d'amines selon les revendications 1 à 7, caractérisé par le fait que l'on utilise en tant que catalyseurs hétérogènes des oxydes cristallins à base d'aluminophosphates à structure de faujasite, façonnés avec un liant et calcinés à des températures de 200 à 600°C.

9. Procédé pour la préparation d'amines selon les revendications 1 à 8, caractérisé par le fait que l'on utilise en tant que catalyseurs hétérogènes des silicoaluminophosphates (SAPOs), en particulier le SAPO-37 ou le FCAPO-37, des métallo-silicoaluminophosphates (MeAPSOs), en particulier le McAPSO-37, des aluminophosphates (AlPOs) contenant une excès d'aluminium, en particulier l'AlPO-37, ou des métallo-aluminophosphates (MeAPOs), en particulier MeAPO-37.

10. Procédé pour la préparation d'amines selon la revendication 9, caractérisé par le fait que l'on utilise en tant que catalyseurs hétérogènes le SAPO-37, le FCAPO-37, l'AIPO-37 ou le MeAPO-37.
